# EUROPEAN PATENT APPLICATION

(11) **EP 1 126 025 A2**
(43) Date of publication of application: **22.08.2001**
(21) Application number: 01201830.5
(22) Date of filing: 25.02.1993
(51) Int. Cl.: C12N 15/11, C07H 21/00, C12Q 1/68, A61K 48/00

(54) **Oligonucleotide modulation of protein kinase C**

(30) Priority: 16.03.1992 US 852852
(62) Divisional of application: 93907417.5
(71) Applicant: ISIS PHARMACEUTICALS, INC., Carlsbad, CA 92008 (US)
(72) Inventor: Bennet, C. Frank, Carlsbad, CA 92008 (US); Dean, Nicholas, Cardiff-by-the-Sea, CA 92007 (US)
(74) Representative: Hallybone, Huw George

(57) **Abstract**

Compositions and methods are provided for the treatment and diagnosis of diseases associated with protein kinase C. Oligonucleotides are provided which are specifically hybridizable with nucleic acids encoding PKC. Oligonucleotides specifically hybridizable with a translation initiation site, 5'-untranslated region or 3'-untranslated region are provided. Oligonucleotides specifically hybridizable with a particular PKC isozyme or set of isozymes are also provided. Methods of treating animals suffering from disease amenable to therapeutic intervention by modulating protein kinase C expression with an oligonucleotide specifically hybridizable with RNA or DNA corresponding to PKC are disclosed.

## Description

### FIELD OF THE INVENTION

This invention relates to therapies, diagnostics, and research reagents for disease states which respond to modulation of the expression of protein kinase C. In particular, this invention relates to antisense oligonucleotides specifically hybridizable with nucleic acids relating to protein kinase C. These oligonucleotides have been found to modulate the expression of protein kinase C. Palliation and therapeutic effect result.

### BACKGROUND OF THE INVENTION

The phosphorylation of proteins plays a key role in the transduction of extracellular signals into the cell. The enzymes, called kinases, which effect such phosphorylations are targets for the action of growth factors, hormones, and other agents involved in cellular metabolism, proliferation and differentiation. One of the major signal transduction pathways involves the enzyme protein kinase C (PKC), which is known to have a critical influence on cell proliferation and differentiation. PKC is activated by diacylglycerols (DAGS), which are metabolites released in signal transduction.

Interest in PKC was stimulated by the finding that PKC is the major, and perhaps only, cellular receptor through which a class of tumor-promoting agents called phorbol esters exert their pleiotropic effects on cells. Gescher et al., *Anti-Cancer Drug Design* **1989**, 4, 93-105. Phorbols capable of tumor production can mimic the effect of DAG in activating PKC, suggesting that these tumor promotors act through PKC and that activation of this enzyme is at least partially responsible for the resulting tumorigenesis. Parker et al., *Science* 1986, 233, 853-866.

Experimental evidence indicates that PKC plays a role in growth control in colon cancer. It is believed that specific bacteria in the intestinal tract convert lipids to DAG, thus activating PKC and altering cell proliferation. This may explain the correlation between high dietary fat and colon cancer. Weinstein, *Cancer Res. (Suppl.)* **1991**, 51, 5080s-5085s. It has also been demonstrated that a greater proportion of the PKC in the colonic mucosa of patients with colorectal cancer is in an activated state compared to that of patients without cancer. Sakanoue et al., *Int. J. Cancer* **1991,** 48, 803-806.

Increased tumorigenicity is also correlated with overexpression of PKC in cultured cells inoculated into nude mice. A mutant form of PKC induces highly malignant tumor cells with increased metastatic potential. Sphingosine and related inhibitors of PKC activity have been shown to inhibit tumor cell growth and radiation-induced transformation *in vivo*. Endo et al., Cancer Research **1991**, 51, 1613-1618; Borek et al., *Proc. Natl. Acad. Sci.* **1991**, 88, 1953-1957. A number of experimental or clinically useful anti-cancer drugs show modulatory effects on PKC. Therefore, inhibitors of PKC may be important cancer-preventive or therapeutic agents. PKC has been suggested as a plausible target for more rational design of conventional anti-cancer drugs. Gescher, A. and Dale, I.L., *Anti-Cancer Drug Design* **1989,** 4, 93-105.

Experiments also indicate that PKC plays an important role in the pathophysiology of hyperproliferative skin disorders such as psoriasis and skin cancer. Psoriasis is characterized by inflammation, hyperproliferation of the epidermis and decreased differentiation of cells. Various studies indicate a role for PKC in causing these symptoms. PKC stimulation in cultured keratinocytes can be shown to cause hyperproliferation. Inflammation can be induced by phorbol esters and is regulated by PKC. DAG is implicated in the involvement of PKC in dermatological diseases, and is formed to an increased extent in psoriatic lesions.

Inhibitors of PKC have been shown to have both antiproliferative and antiinflammatory effects *in vitro.* Some antipsoriasis drugs, such as cyclosporine A and anthralin, have been shown to inhibit PKC. Inhibition of PKC has been suggested as a therapeutic approach to the treatment of psoriasis. Hegemann, L. and G. Mahrle, Pharmacology of the Skin, H. Mukhtar, Ed., CRC Press, Boca Raton, FL, 1992, p. 357-368.

PKC is not a single enzyme, but a family of enzymes. At the present time at least seven isoforms (isozymes) of PKC have been identified: isoforms α, β, and γ have been purified to homogeneity, and isoforms δ, ∈, ζ and η have been identified by molecular cloning. These isozymes have distinct patterns of tissue and organ localization (see Nishizuka, *Nature* **1988**, 334, 661-665 for review) and may serve different physiological functions. For example, PKC-γ seems to be expressed only in the central nervous system. PKC-α and -β are expressed in most tissues, but have different patterns of expression in different cell types. For example, both PKC-a and PKC-β are expressed in, and have been purified from, human epidermis. While PKC-α has been detected mainly in keratinocytes of the basal layers of the epidermis, PKC-β is found mainly in the middle layers of the epidermis and Langerhans cells. PKC-η has been found predominantly in the skin and lungs, with levels of expression much higher in these tissues than in the brain. This is in contrast to other members of the PKC family which tend to be most abundantly expressed in the brain. Osada et al., *J. Biol. Chem.* **1990,** 265, 22434-22440. While the PKC isozymes listed here are preferred for targeting by the present invention, other isozymes of PKC are also comprehended by the present invention.

It is presently believed that different PKC isozymes may be involved in various disease processes depending on the organ or tissue in which they are expressed. For example, in psoriatic lesions there is an alteration in the ratio between PKC-α and PKC-β, with preferential loss of PKC-β compared to normal skin (Hegemann, L. and G. Mahrle, Pharmacology of the Skin, H. Mukhtar, Ed., CRC Press, Boca Raton, FL, 1992, p. 357-368.

Although numerous compounds have been identified as PKC inhibitors (see Hidaka and Hagiwara, *Trends in Pharm. Sci.* **1987**, 8, 162-164 for review), none has been found which inhibits PKC specifically. While the quinoline sulfonamide derivatives such as 1-(5-isoquinolinesulfonyl)-2-methylpiperazine (H-7) inhibit PKC at micromolar concentrations, they exhibit similar enzyme inhibition kinetics for PKC and the cAMP-dependent and cGMP-dependent protein kinases. Staurosporine, an alkaloid product of *Streptomyces* sp., and its analogs, are the most potent *in vitro* inhibitors of PKC identified to date. However, they exhibit only limited selectivity among different protein kinases. Gescher, *Anti-Cancer Drug Design* **1989**, 4, 93-105. Thus there has been a failure of others to inhibit PKC specifically. There is also a desire to inhibit specific PKC isozymes, both as a research tool and as treatment for diseases which may be associated with particular isozymes.

### OBJECTS OF THE INVENTION

It is a principal object of the invention to provide therapies for neoplastic, hyperproliferative, inflammatory and other disease states associated with protein kinase C.

Another object of the invention is to provide selective therapies for diseases associated with particular isozymes of protein kinase C.

It is a further object of the invention to provide antisense oligonucleotides which are capable of modulating the expression of protein kinase C.

Another object of the invention is to provide antisense oligonucleotides which are capable of selectively modulating the expression of particular isozymes of protein kinase C.

Yet another object is to provide means for diagnosis of diseases associated with protein kinase C.

A further object of the invention is to provide means for differential diagnosis of diseases associated with particular isozymes of protein kinase C.

A still further object of the invention is to provide research tools for the study of the effects of protein kinase C expression and diseases associated therewith.

An additional object of the invention is to provide research tools for the study of the effects of expression of particular isozymes of protein kinase C and diseases associated therewith.

These and other objects of this invention will become apparent from a review of the instant specification.

### BRIEF DESCRIPTION OF THE DRAWINGS

Figure 1(a) and 1(b) are graphical depictions of the effects on PKC expression of antisense oligonucleotides hybridizable with PKC-α. Oligonucleotides are arranged by PKC target region, 5' to 3'.
Figure 2 is a line graph showing dose-dependent antisense oligonucleotide inhibition of PKC-α protein synthesis, expressed as percent of control (no oligonucleotide). ▼ = ISIS 3520; ■ = ISIS 3522; ▲ = ISIS 3527; ▼ = ISIS 4985.
Figure 3 is a line graph showing the dose-dependent inhibition of PKC-α protein synthesis by ISIS 4649, the 2'-O-methyl version of ISIS 3522 (both are phosphorothioates). ▼ = ISIS 4632; ● = ISIS 4636; ■ = ISIS 4649; ▲ = ISIS 4648.
Figure 4 is a bar graph showing decrease in levels of PKC-α mRNA transcripts after antisense oligonucleotide treatment. The hatched bars represent the 8.5 kb transcript and the white bars represent the 4.0 kb transcript.
Figure 5 is a line graph showing antisense oligonucleotide inhibition of A549 cell proliferation. ■ = 4559;◆ = 4985;● = 3521; ▲ = 3527.
Figure 6 is a series of line graphs showing inhibition of PKCα mRNA levels by 2'-O-methyl phosphorothioate oligonucleotides having 8-deoxy gaps compared to full-deoxy phosphorothioate oligonucleotides having the same sequence.
Figure 6A shows data for SEQ ID NO: 2 (5357 is gapped oligonucleotide, 3521 is full deoxy). Figure 6B shows data for SEQ ID NO: 3 (5352 is gapped oligonucleotide, 3522 is full deoxy). Figure 6C shows data for SEQ ID NO: 5 (5240 is gapped oligonucleotide, 3527 is full deoxy).
Figure 7 is a line graph showing the relationship between deoxy gap length (in a 2'-O-methyl oligonucleotide) and reduction of PKCα mRNA levels by antisense oligonucleotides having SEQ ID NO: 2, SEQ ID NO: 3 and SEQ ID NO: 5.

### SUMMARY OF THE INVENTION

In accordance with the present invention, oligonucleotides are provided that are specifically hybridizable with DNA or RNA deriving from the gene that encodes PKC. The oligonucleotide comprises nucleotide units sufficient in identity and number to effect such specific hybridization. This relationship is commonly denominated as "antisense". In one preferred embodiment, the oligonucleotides are specifically hybridizable with the translation initiation codon of the gene, and preferably comprise a sequence CAT. In another preferred embodiment, the oligonucleotides are specifically hybridizable with the 5'-untranslated or 3'-untranslated regions of the gene. In yet another preferred embodiment, oligonucleotides are provided that are specifically hybridizable with DNA or mRNA encoding a particular PKC isozyme or a particular set of PKC isozymes. Such oligonucleotides are conveniently and desirably presented in a pharmaceutically acceptable carrier.

In accordance with other preferred embodiments, the oligonucleotides are formulated such that at least one of the linking groups between nucleotide units of the oligonucleotide comprises a sulfur-containing species such as a phosphorothioate moiety. In still other preferred embodiments, the oligonucleotides are formulated such that at least one of the nucleotides is modified at the 2' position of the sugar. Some examples of such preferred modifications are 2'-O-alkyl and 2'-fluoro modifications.

Other aspects of the invention are directed to methods for modulating the expression of PKC or of a particular PKC isozyme or set of isozymes in cells or tissues. Additional aspects of the invention are directed to methods of detection in cells or tissues of the DNA or RNA that encodes PKC and specific detection in cells or tissues of RNA or DNA that encodes particular PKC isozymes. Such methods comprise contacting cells or tissues suspected of containing said gene with oligonucleotides in accordance with the invention in order to interfere with the effect of or to detect said RNA or DNA.

Other aspects of the invention are directed to methods for diagnostics and therapeutics of animals suspected of having a disease associated with PKC or one of its isozymes. Such methods comprise contacting the animal or cells or tissues or a bodily fluid from the animal with oligonucleotides in accordance with the invention in order to modulate the expression of PKC, to treat conditions associated with PKC, or to effect a diagnosis thereof.

### DETAILED DESCRIPTION OF THE INVENTION

Antisense oligonucleotides hold great promise as therapeutic agents for the treatment of many human diseases. Oligonucleotides specifically bind (hybridize) to the complementary sequence of DNA, pre-mRNA or mature mRNA, as defined by Watson-Crick base pairing, interfering with the flow of genetic information from DNA to protein. The properties of antisense oligonucleotides which make them specific for their target sequence also make them extraordinarily versatile. Because antisense oligonucleotides are long chains of monomeric units, they may be readily synthesized for any target RNA sequence. Numerous recent studies have documented the utility of antisense oligonucleotides as biochemical tools for studying target proteins. Rothenberg et al., J. *Natl. Cancer Inst.* **1989,** 81, 1539-1544; Zon, G., *Pharmaceutical Res.* **1988,** 5, 539-549. Because of recent advances in oligonucleotide chemistry and synthesis of nuclease resistant oligonucleotides which exhibit enhanced cell uptake, it is now possible to consider the use of antisense oligonucleotides as a novel form of therapeutics.

Antisense oligonucleotides offer an ideal solution to the problems encountered in prior art approaches. They can be designed to selectively inhibit a given isozyme or particular set of isozymes, or to inhibit all members of a given family of isozymes. They avoid non-specific mechanisms such as free radical scavenging. A complete understanding of enzyme mechanism is not needed to design specific inhibitors.

Current agents which modulate the activity or metabolism of protein kinase C exhibit many unacceptable side effects due to their lack of specificity, or they exhibit only limited effectiveness in inhibiting the enzyme. The instant invention circumvents problems encountered by prior workers by modulating the production of the enzyme, rather than inhibiting the enzyme directly, to achieve the therapeutic effect. In the instant invention, the oligonucleotide is designed to bind directly to mRNA or to a gene, ultimately modulating the amount of PKC protein made from the gene.

In the context of this invention, the term "oligonucleotide" refers to a polynucleotide formed from naturally occurring bases and pentofuranosyl groups joined by native phosphodiester bonds. This term effectively refers to naturally occurring species or synthetic species formed from naturally occurring subunits or their close homologs. The term "oligonucleotide" may also refer to moieties which function similarly to naturally occurring oligonucleotides but which have non-naturally occurring portions. Thus, oligonucleotides may have altered sugar moieties or inter-sugar linkages. Exemplary among these are the phosphorothioates and other sulfur-containing species such as phosphorodithioates which are known for use in the art. In accordance with some preferred embodiments, at least one of the phosphodiester bonds of the oligonucleotide have been substituted with a structure which functions to enhance the ability of the compositions to penetrate into the region of cells where the RNA or DNA whose activity to be modulated is located. It is preferred that such substitutions comprise phosphorothioate bonds, methyl phosphonate bonds, short chain alkyl or cycloalkyl structures, or heteroatom-substituted short chain alkyl structures. Most preferred are those with CH₂-NH-O-CH₂, CH₂-N(CH₃)-O-CH₂, CH₂-O-N(CH₃)-CH₂, CH₂-N(CH₃)-N(CH₃)-CH₂ and O-N(CH₃)-CH₂-CH₂ backbones (where phosphodiester is O-P-O-CH₂). Also preferred are oligonucleotides having morpholino backbone structures. Summerton, J.E. and Weller, D.D., U.S. Patent No: 5,034,506. In other preferred embodiments, such as the protein-nucleic acid (PNA) backbone, the phosphodiester backbone of the oligonucleotide may be replaced with a polyamide backbone, the bases being bound directly or indirectly to the aza nitrogen atoms of the polyamide backbone. P.E. Nielsen, M. Egholm, R.H. Berg, O. Buchardt, *Science* **1991**, 254, 1497. In accordance with other preferred embodiments, the phosphodiester bonds are substituted with other structures which are, at once, substantially non-ionic and non-chiral, or with structures which are chiral and enantiomerically specific. Persons of ordinary skill in the art will be able to select other linkages for use in practice of the invention.

Oligonucleotides may also include species which include at least one modified base form. Thus, purines and pyrimidines other than those normally found in nature may be so employed. Similarly, modifications on the pentofuranosyl portion of the nucleotide subunits may also be effected, as long as the essential tenets of this invention are adhered to. Examples of such modifications are 2'-O-alkyl- and 2'-halogen-substituted nucleotides. Some specific examples of modifications at the 2' position of sugar moieties which are useful in the present invention are OH, SH, SCH₃, F, OCH₃, OCN, O(CH₂)ₙNH₂ or O(CH₂)ₙCH₃ where n is from 1 to about 10, and other substituents having similar properties. Sugar mimetics such as cyclobutyls or other carbocycles may also be used in place of the pentofuranosyl group. Chimeric oligonucleotides having two or more chemically distinct regions are also useful in the present invention. Specific examples of such chimeric oligonucleotides are those which are modified at the 2' position except for a "deoxy gap" of one or more deoxynucleotides. The "deoxy gap" may be centered in the molecule or may be located at or near either end. oligonucleotides with regions of distinct backbone chemistries are also examples of chimeric oligonucleotides.

Such oligonucleotides are best described as being functionally interchangeable with natural oligonucleotides (or synthesized oligonucleotides along natural lines), but having one or more differences from natural structure. All such oligonucleotides are comprehended by this invention so long as they function effectively to hybridize with the PKC RNA. The oligonucleotides in accordance with this invention preferably comprise from about 5 to about 50 nucleotide units. It is more preferred that such oligonucleotides comprise from about 8 to 30 nucleotide units, and still more preferred to have from about 12 to 25 nucleotide units. As will be appreciated, a nucleotide unit is a base-sugar combination suitably bound to an adjacent nucleotide unit through phosphodiester or other bonds.

The oligonucleotides used in accordance with this invention may be conveniently and routinely made through the well-known technique of solid phase synthesis. Equipment for such synthesis is sold by several vendors including Applied Biosystems. Any other means for such synthesis may also be employed; the actual synthesis of the oligonucleotides is well within the talents of the routineer. It is also well known to use similar techniques to prepare other oligonucleotides such as the phosphorothioates and alkylated derivatives.

In accordance with this invention, persons of ordinary skill in the art will understand that messenger RNA includes not only the information to encode a protein using the three letter genetic code, but also associated ribonucleotides which form a region known to such persons as the 5'-untranslated region, the 3'-untranslated region, the 5' cap region and intron/exon junction ribonucleotides. Thus, oligonucleotides may be formulated in accordance with this invention which are targeted wholly or in part to these associated ribonucleotides as well as to the informational ribonucleotides. In preferred embodiments, the oligonucleotide is specifically hybridizable with a transcription initiation site, a translation initiation site, a 5' cap region, an intron/exon junction, coding sequences or sequences in the 5'- or 3'-untranslated region.

The oligonucleotides of this invention are designed to be hybridizable with messenger RNA derived from the PKC gene. Such hybridization, when accomplished, interferes with the normal roles of the messenger RNA to cause a modulation of its function in the cell. The functions of messenger RNA to be interfered with include all vital functions such as transcription of the RNA from DNA, translocation of the RNA to the site for protein translation, actual translation of protein from the RNA, splicing of the RNA to yield one or more mRNA species, and possibly even independent catalytic activity which may be engaged in by the RNA. The overall effect of such interference with the RNA function is to modulate expression of the PKC gene.

The oligonucleotides of this invention can be used in diagnostics, therapeutics, prophylaxis, and as research reagents and kits. Since the oligonucleotides of this invention hybridize to the PKC gene and its mRNA, sandwich and other assays can easily be constructed to exploit this fact. Furthermore, since the oligonucleotides of this invention hybridize specifically to particular isozymes of the PKC mRNA, such assays can be devised for screening of cells and tissues for particular PKC isozymes. Such assays can be utilized for diagnosis of diseases associated with various PKC forms. Provision of means for detecting hybridization of oligonucleotide with the PKC gene can routinely be accomplished. Such provision may include enzyme conjugation, radiolabelling or any other suitable detection systems. Kits for detecting the presence or absence of PKC may also be prepared.

For therapeutic or prophylactic treatment, oligonucleotides are administered in accordance with this invention. oligonucleotides may be formulated in a pharmaceutical composition, which may include carriers, thickeners, diluents, buffers, preservatives, surface active agents and the like in addition to the oligonucleotide. Pharmaceutical compositions may also include one or more active ingredients such as antimicrobial agents, antiinflammatory agents, anesthetics, and the like in addition to oligonucleotides.

The pharmaceutical composition may be administered in a number of ways depending on whether local or systemic treatment is desired, and on the area to be treated. Administration may be done topically (including ophthalmically, vaginally, rectally, intranasally), orally, by inhalation, or parenterally, for example by intravenous drip or subcutaneous, intraperitoneal or intramuscular injection.

Formulations for topical administration may include ointments, lotions, creams, gels, drops, suppositories, sprays, liquids and powders. Conventional pharmaceutical carriers, aqueous, powder or oily bases, thickeners and the like may be necessary or desirable. Coated condoms may also be useful.

Compositions for oral administration include powders or granules, suspensions or solutions in water or non-aqueous media, capsules, sachets, or tablets. Thickeners, flavorings, diluents, emulsifiers, dispersing aids or binders may be desirable.

Formulations for parenteral administration may include sterile aqueous solutions which may also contain buffers, diluents and other suitable additives.

Dosing is dependent on severity and responsiveness of the condition to be treated, but will normally be one or more doses per day, with course of treatment lasting from several days to several months or until a cure is effected or a diminution of disease state is achieved. Persons of ordinary skill can easily determine optimum dosages, dosing methodologies and repetition rates.

The following examples illustrate the present invention and are not intended to limit the same.

### EXAMPLES

### Example 1 Oligonucleotide synthesis

Unmodified DNA oligonucleotides are synthesized on an automated DNA synthesizer (Applied Biosystems model 380B) using standard phosphoramidite chemistry with oxidation by iodine. β-Cyanoethyldiisopropyl-phosphoramidites are purchased from Applied Biosystems (Foster City, CA). For phosphorothioate oligonucleotides, the standard oxidation bottle is replaced by a 0.2 M solution of 3H-1,2-benzodithiole-3-one 1,1-dioxide in acetonitrile for the stepwise thiation of the phosphite linkages. The thiation cycle wait step is increased to 68 seconds and is followed by the capping step.

After cleavage from the controlled pore glass column (Applied Biosystems) and deblocking in concentrated ammonium hydroxide at 55°C for 18 hours, the oligonucleotides are purified by precipitation twice out of 0.5 M NaCl with 2.5 volumes ethanol. Analytical gel electrophoresis is accomplished in 20% acrylamide, 8 M urea, 45 mM Tris-borate buffer, pH 7.0.

### Example 2 Cell culture and treatment with phorbol esters and oligonucleotides targeted to PKC-α

PKC protein half-lives have been reported to vary from 6.7 hours to over 24 hours. Young et al., Biochem. J. **1987**, 244, 775-779; Ballester et al., *J. Biol. Chem.* **1985**, 260, 15194-15199. These long half-lives make inhibiting steady-state levels of PKC-α an unwieldy approach when screening antisense oligonucleotides, due to the long incubation times which would be required. The ability of phorbol esters to reversibly lower intracellular levels of PKC has therefore been exploited. Treatment of cells with phorbol esters causes an initial activation of kinase activity, followed by a down-regulation of PKC. For PKC-a this down-regulation has been shown to be a direct consequence of an increased rate of proteolysis of the kinase with no apparent change in synthetic rate.

It was initially determined that in human lung carcinoma (A549) cells, treatment with the phorbol ester 12,13-dibutyrate (PDBu), using a modification of the method of Krug et al., *J*. *Biol. Chem.* **1987**, 262, 11852-11856, did indeed lower cellular levels of PKC-α, without affecting PKC-α mRNA levels, and that this effect was reversible. The basis of the assay to screen for potency of oligonucleotides targeting PKC-α is to initially lower PKC-α protein levels by chronic treatment with PDBu, remove PDBu by extensively washing the cells (hence allowing the cells to synthesize fresh PKC-α protein), and incubate the cells with oligonucleotides intended to inhibit the resynthesis of new PKC-α protein.

A549 cells (obtained from the American Type Culture Collection, Bethesda MD) were grown to confluence in 6-well plates (Falcon Labware, Lincoln Park, NJ) in Dulbecco's modified Eagle's medium (DME) containing 1 g glucose/liter and 10% fetal calf serum (FCS, Irvine Scientific, Santa Ana, CA).

Cells were treated with 500 nM PDBu (Sigma Chem. Co., St. Louis, MO) for 12-16 hours (overnight). Cells were then washed three times in DME at 37°, and 1 ml DMA containing 20 *µ*l DOTMA (Lipofectin reagent, BRL, Bethesda, MD) was added. Phosphorothioate oligonucleotides were added to a concentration of 1 *µ*M and the cells were incubated for a further 4 hours at 37°C.

Cells were washed once in 3 ml DME containing 0.1 mg/ml BSA and a further 2 ml DME containing 0.1 mg/ml BSA was added. Phosphorothioate oligonucleotides (1 *µ*M) were added and the cells were incubated at 37°C for 24 hours.

Cells were washed three times in phosphate-buffered saline (PBS) and cellular proteins were extracted in 120 *µ*l sample buffer (60 mM Tris pH 6.8, 2% SDS, 10% glycerol, 10 mM dithiothreitol) and boiled for 5 minutes. Intracellular levels of PKC-α protein were determined by immunoblotting.

The oligonucleotides tested in this assay are presented in Table 1. Sequence data are from the cDNA sequence published by Finkenzeller et al., *Nucl. Acids Res.* **1990,** 18, 2183; the sequence numbers given under the oligonucleotides are relative to the first residue to be sequenced on the cDNA, which is 28 residues upstream of the ATG start codon.

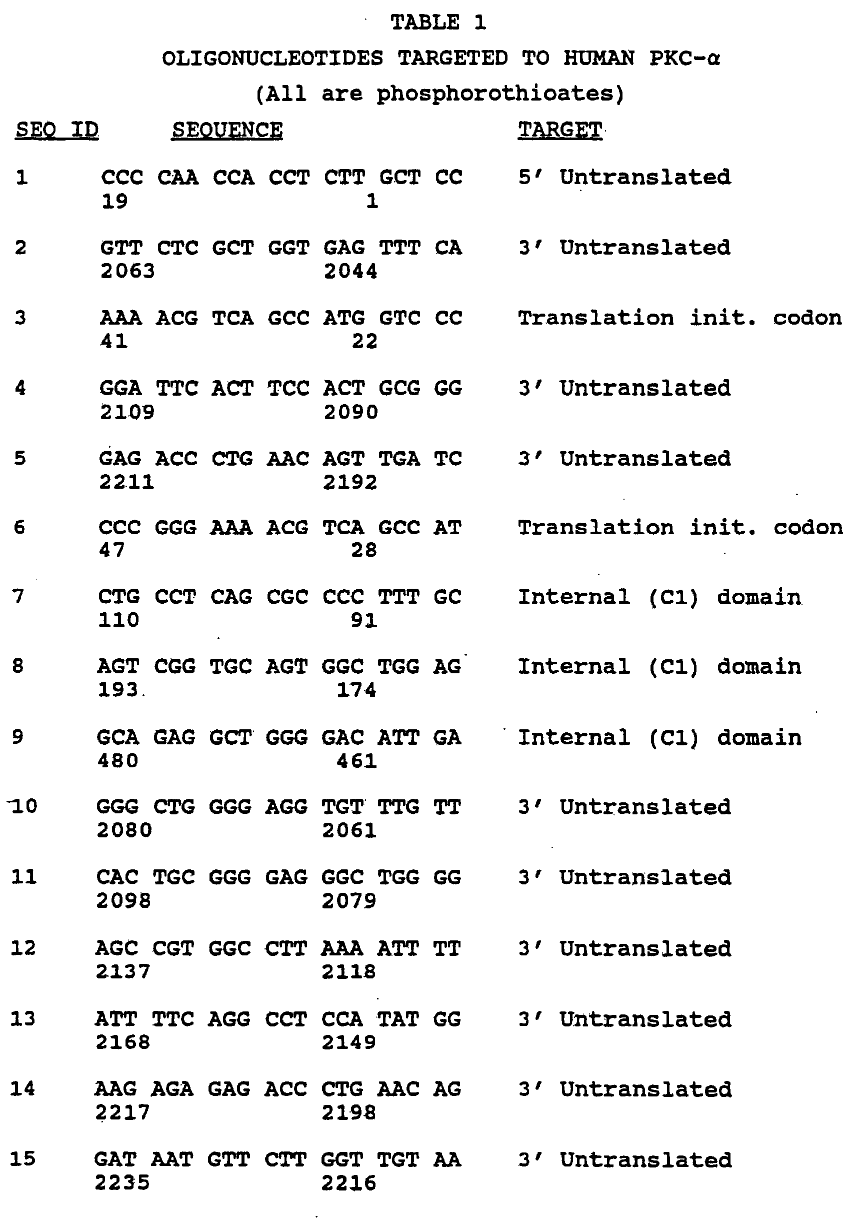

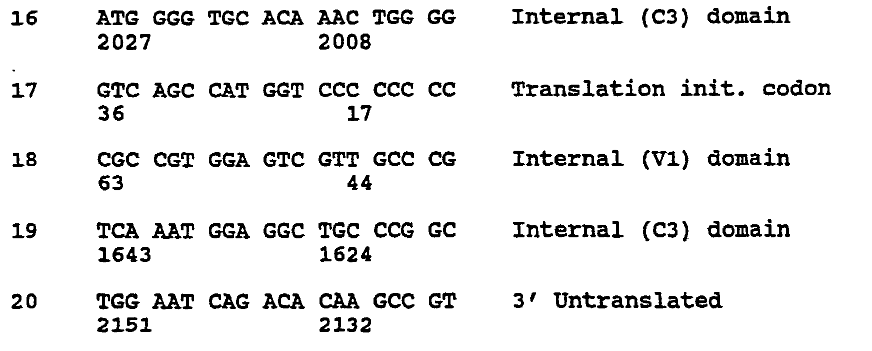

### Example 3 Immunoblot assay for PKC expression

Cell extracts were electrophoresed on 10% SDS-PAGE mini-gels. The resolved proteins were transferred to Immobilon-P membrane (Millipore, Bedford MA) by electrophoretic transfer and the membrane was blocked for 60 minutes in TBS (Tris-HCl pH 7.4, 150 mM NaCl) containing 5% nonfat milk. The membrane was then incubated for 16 hours at 4°C with monoclonal antibodies raised against PKC-α (UBI, Lake Placid NY) diluted to 0.2 *µ*g/ml in TBS containing 0.2% nonfat milk. This was followed by three washes in TBS plus 0.2% nonfat milk. The membrane was then incubated for one hour with ¹²⁵I-labelled goat anti-mouse secondary antibody (ICN Radiochemicals, Irvine CA). Membranes were then washed extensively in TBS plus 0.2% nonfat milk. Bands were visualized and quantitated using a Phosphorimager (Molecular Dynamics, Sunnyvale, CA). PKC-α appears as a single band with a molecular weight of 80 kD.

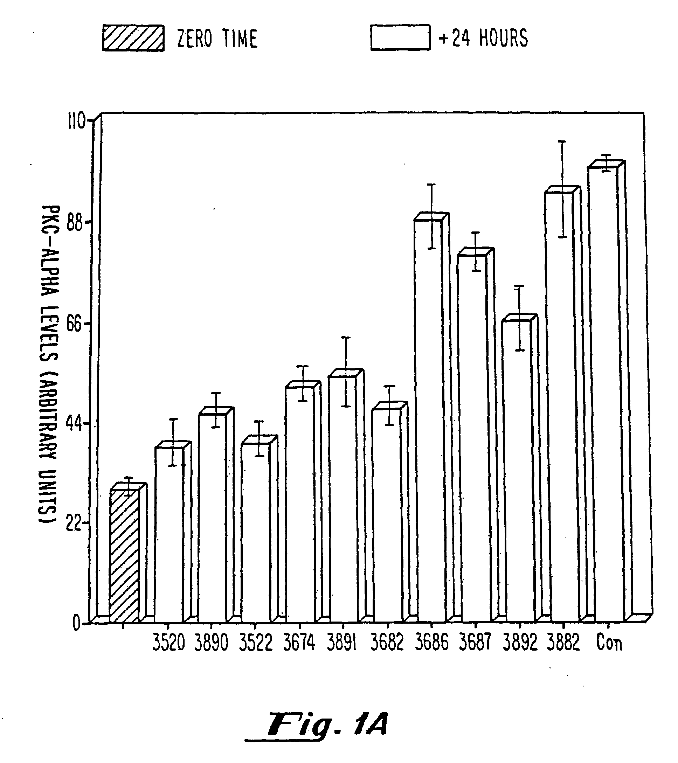

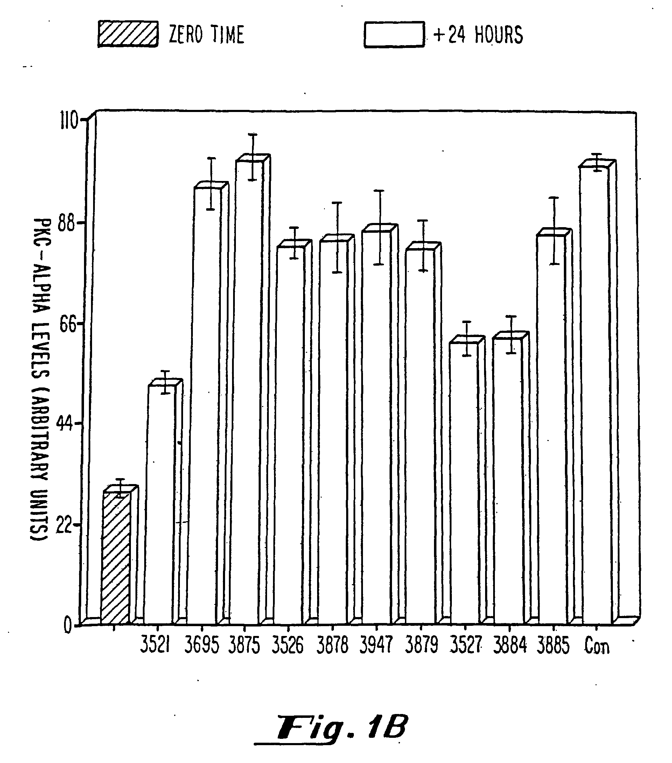

Each oligonucleotide was tested three times, in triplicate, and the results of the experiments were normalized against percentage of protein present as compared to cells which were not treated with oligonucleotide (Figure 1). The five most effective oligonucleotides target the AUG start codon and regions slightly upstream and downstream from it (oligos 1, 3, 17, 7, 6). The next most effective oligonucleotides are targeted toward the 3' untranslated region of the RNA (oligos 2, 5, 14).

### Example 4 Other isozymes of PKC

It was found that the most effective sequences for antisense targeting of human PKCα are those surrounding the translation initiation codon and the 3' untranslated region. It is believed that these sequences would also be effective targets for oligonucleotides directed against other isozymes of PKC. The other isozymes of human PKC for which sequence data are available are PKC-β (types I and II), PKC-γ (partial sequence) and PKC-η. Antisense oligonucleotides which are likely to be effective inhibitors of PKC are identified below. These oligonucleotides are synthesized as in Example 1, and can be screened as in Examples 2 and 3, using appropriate antibodies where available. Alternatively, a reporter gene assay system can be established, transiently co-expressing the desired isozyme of PKC with luciferase under the influence of the TPA-responsive enhancer or other suitable promoter. PKC expression is then assayed by measuring luciferase activity using standard procedures: luciferase is extracted from cells by lysis with the detergent Triton X-100, as described by Greenberg, M.E., in Current Protocols in Molecular Biology, F.M. Ausubel, R. Brent, R.E. Kingston, D.D. Moore, J.A. Smith, J.G. Seidman and K. Strahl, Eds., John Wiley and Sons, NY (1987). A Dynatech ML1000 luminometer is used to measure peak luminescence upon addition of luciferin (Sigma) to 625 *µ*M.

### PKC-β, types I and II

Sequence data are from Kubo et al., *FEBS Lett.* **1987,** 223, 138-142; sequences are numbered from the first 5' base sequenced on the cDNA. PKC-β types I and II are the result of alternative mRNA splicing of a single gene product. This results in proteins with identical amino termini (5' end of the mRNA); however, there is sequence divergence in the carboxy termini (3' end of the mRNA). The following oligonucleotides, targeted to the translation initiation codon, are expected to modulate expression of both PKC-β types I and II:

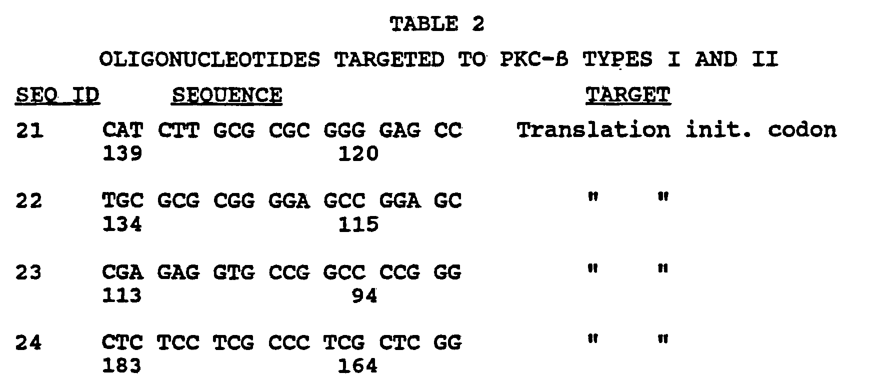

The following antisense oligonucleotides are targeted to the 3'-untranslated region of PKC-β type I only:

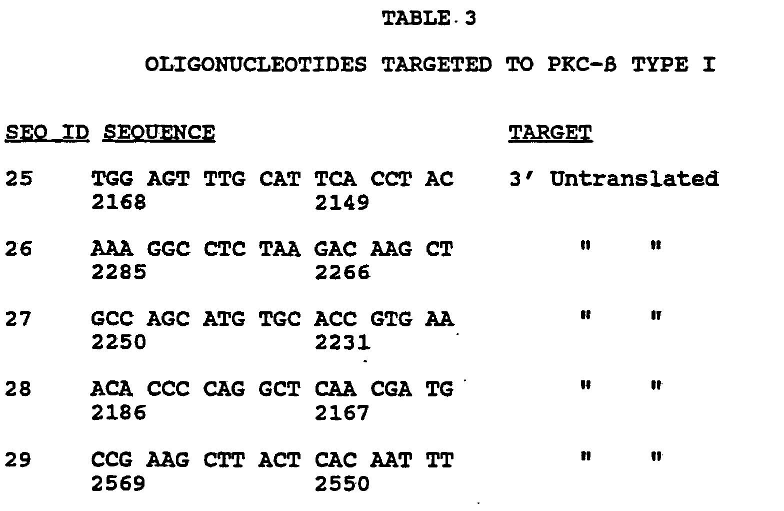

The following antisense oligonucleotides are targeted to the 3'-untranslated region of PKC-β Type II only:

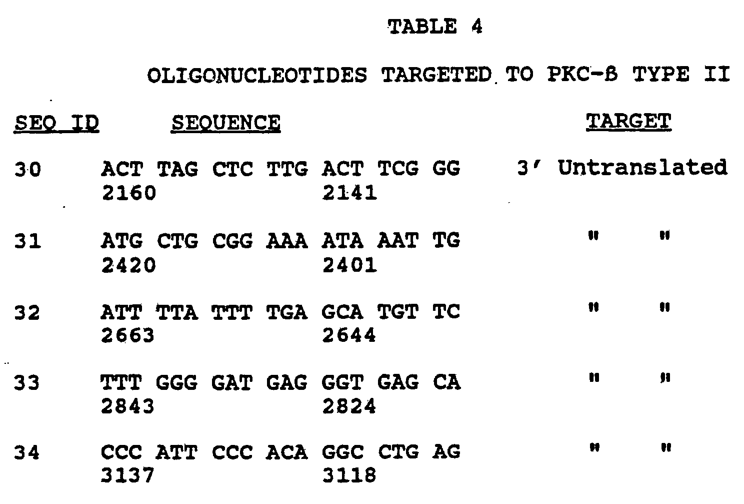

### PKC-γ:

Sequence data are from Coussens et al., *Science* **1986**, 233, 859-866; sequences are numbered from the first 5' base sequenced in the cDNA. The full sequence is not available: the extreme 3' end of the open reading frame and the 3' untranslated region are missing. Consequently these regions are not presently available as antisense targets.

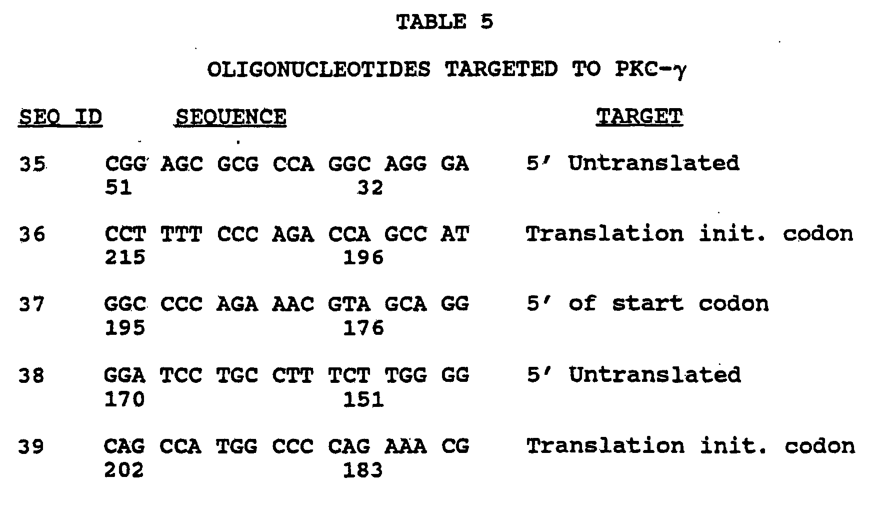

### PKC-η:

Sequence data for PKC-η are from Bacher and colleagues [Bacher et al., *Mol. Cell. Biol.* **1991,** 11, 126-133]. They assign their isozyme the name PKC-L; however the sequence is almost identical to that of mouse PKC-η, so the latter nomenclature is used here for consistency. Sequences are numbered from the first 5' base sequenced in the cDNA.

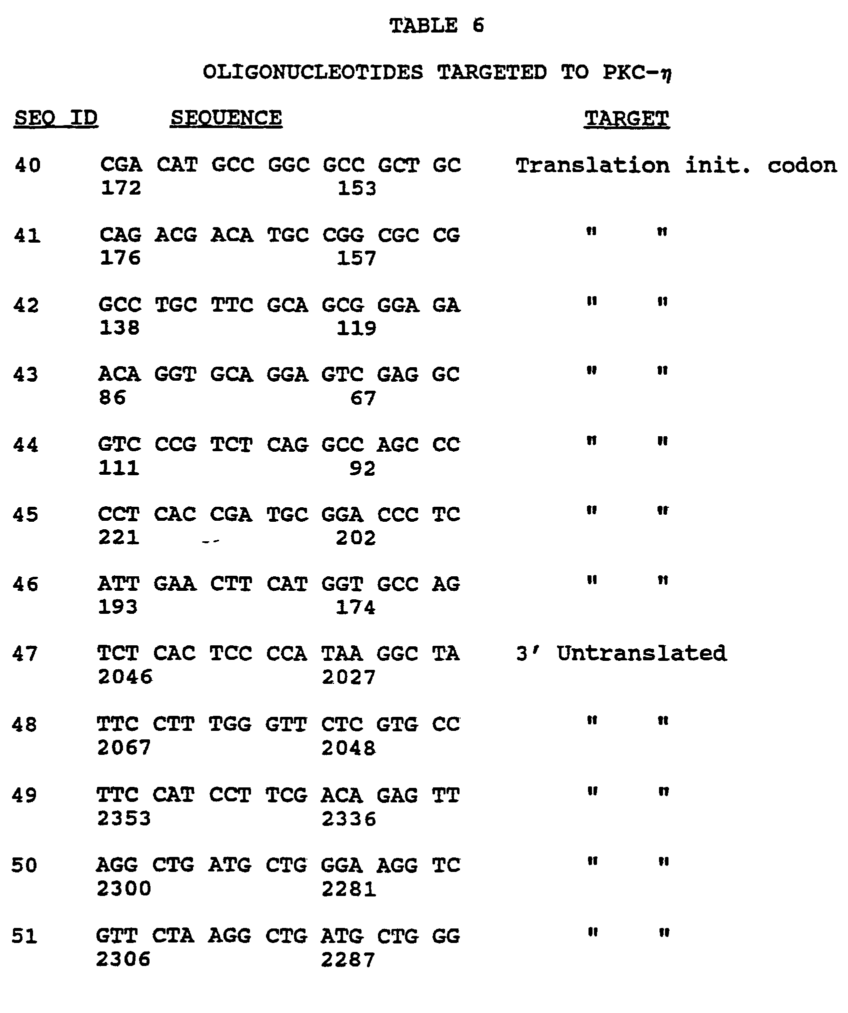

### Example 5 Oligonucleotide inhibition of PKC is dose-dependent

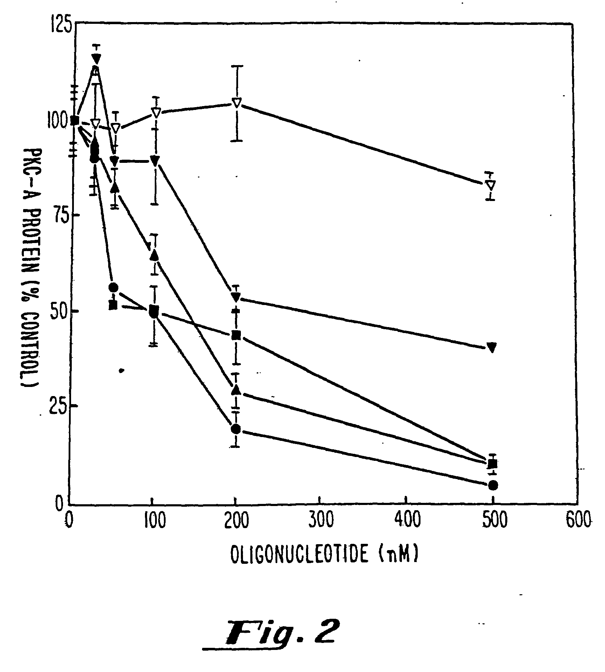

Four oligonucleotides shown in Figure 1 to be active against PKC-α were characterized further. Dose response studies of ISIS 3520 (SEQ ID NO:1), 3521 (SEQ ID NO:2), 3522 (SEQ ID NO:3) and 3527 (SEQ ID NO:5) using the immunoblotting assay described in Example 3 demonstrated that all had dose-dependent activity against PKC-α protein expression. ISIS 3521, 3522 and 3527 had IC₅₀ values of 100-200 nM, and all maximally inhibited PKC expression at 500 nM. This is shown in Figure 2. ISIS 4985 (SEQ ID NO: 52), a scrambled control oligonucleotide having the same base composition as ISIS 3527, was without effect.

### Example 6 Synthesis of 2'-O-methyl phosphorothioate oligonucleotides

2'-O-methyl phosphorothioate oligonucleotides were synthesized using 2'-O-methyl β-cyanoethyldiisopropyl-phosphoramidites (Chemgenes, Needham MA) and the standard cycle for unmodified oligonucleotides, except the wait step after pulse delivery of tetrazole and base was increased to 360 seconds. The 3'-base used to start the synthesis was a 2'-deoxyribonucleotide.

### Example 7 Effect of 2'-O-methylated version of ISIS 3522 on PKC-α protein synthesis

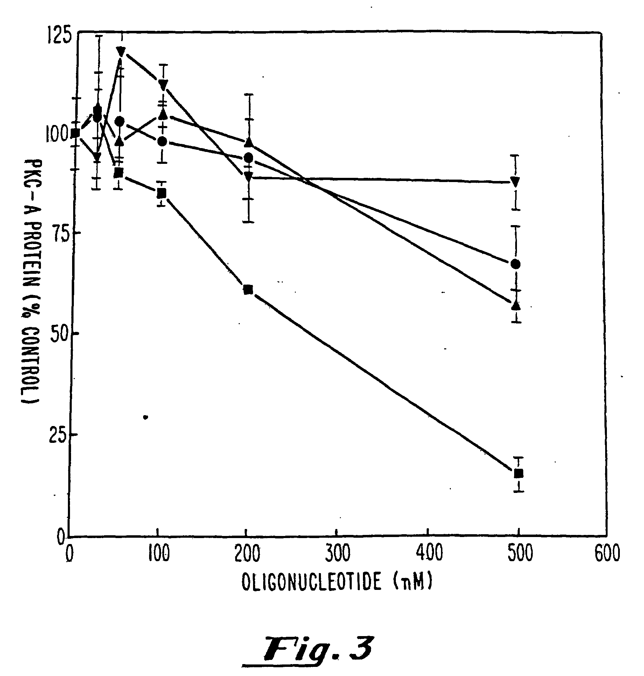

A uniformly 2'-O-methyl modified phosphorothioate oligonucleotide, ISIS 4649, having the same sequence as the phosphorothioate oligonucleotide ISIS 3522 (SEQ ID NO:3), was able to inhibit PKC protein synthesis (demonstrated by immunoblotting as described in Example 3), with an IC₅₀ of less than 300 nM. This is shown in Figure 3.

### Example 8 Effect of oligonucleotides on PKC-α mRNA expression

To determine the effects of oligonucleotides on PKC-α mRNA levels, A549 cells were treated with oligonucleotides at the indicated concentration in the presence of cationic liposomes for four hours. Total cellular RNA was isolated from cells by lysis in 4M guanidinium isothiocyanate followed by a cesium chloride gradient. Total RNA (15-30 *µ*g) was resolved on 1.2% agarose gels containing 1.1% formaldehyde and transferred to nylon membranes. The membranes were then hybridized with bovine PKC-α cDNA obtained from the ATCC (Bethesda, MD). The cDNA was ³²P radiolabelled with **[α-**^{**32**}**P]dCTP** by random primer labeling using a commercially available kit (Promega) according to the manufacturer's instructions. The filters were hybridized for 60 minutes in Quikhyb solution (Stratagene, San Diego, CA) at 68°C. This was followed by two low stringency washes (2x SSC/0.1% SDS) at room temperature and two high stringency washes (0.1x SSC/0.1% SDS at 60°C. Hybridizing bands were visualized and quantitated using a phosphorimager. The blots were then stripped of radioactivity by boiling and reprobed with a ³²P-labeled glycerol-3-phosphate dehydrogenase (G3PDH) probe (Clontech) to confirm equal RNA loading.

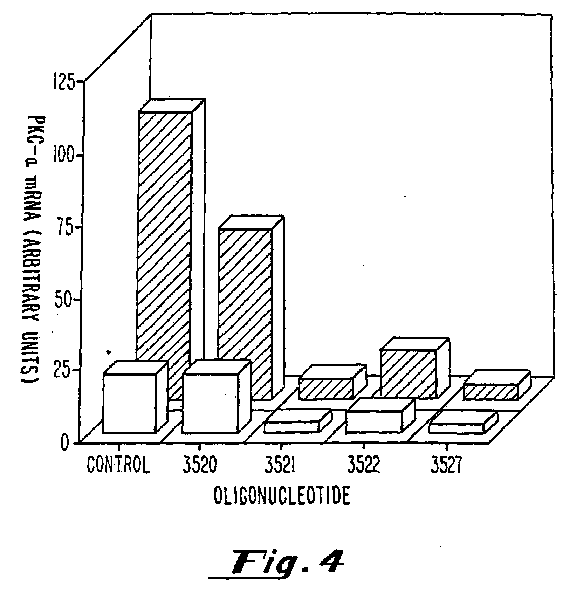

Northern analysis of total RNA from A549 cells using the PKC-α-specific cDNA probe revealed two major hybridizing transcripts approximately 8.5 kb and 4.0 kb in size. Typically these are expressed in a two-to-one ratio with the larger transcript predominating. When A549 cells were treated with antisense oligonucleotides at 500 nM for four hours in the presence of DOTMA and then incubated for an additional 20 hours, levels of both transcripts were decreased. This is shown in Figure 4. The greatest decrease was seen with oligonucleotides ISIS 3521 (SEQ ID NO:2) and 3527 (SEQ ID NO:5), both specifically hybridizable with 3'-untranslated sequences. These reduced amounts of PKC-α mRNA by 90-95%. ISIS 3522. (SEQ ID NO:3), targeted to the translation start codon, decreased PKC-α mRNA levels by 80%, and ISIS 3520 (SEQ ID NO: 1) decreased PKC mRNA by 40%. All oligonucleotides had IC₅₀ values of approximately 200 nM for PKC mRNA reduction. Scrambled control oligonucleotide showed no effect at this concentration.

The kinetics of antisense oligonucleotide decrease in PKC mRNA levels were similar for the four oligonucleotides tested. Within four hours of oligonucleotide addition, 60-70% of maximal PKC RNA reduction occurred, and maximal reduction occurred 12-24 hours after oligonucleotide addition.

### Example 9 Specific inhibition of PKC-α by antisense oligonucleotides

A549 cells were shown to normally express PKC-δ, -∈ and - ζ in addition to PKC-α. The specificity of inhibition of the PKC-α isozyme by antisense oligonucleotides targeted to this isozyme was demonstrated in these cells by immunoblotting after treatment with ISIS 3521 (SEQ ID NO:2, ISIS 3522 (SEQ ID NO:3) or their scrambled control oligonucleotides, ISIS 4559 (SEQ ID NO: 53) and 4608 (SEQ ID NO: 54). A549 cells were treated for four hours with oligonucleotide (either 400 nM or 300 nM) and DOTMA, washed, and allowed to recover for 48 hours. Cells were then treated again with oligonucleotide (250 nM) and DOTMA, washed and allowed to recover for a further 20 hours. Cell extracts were electrophoresed, transferred to membrane and blocked as described in Example 3. The membrane was then treated overnight with either of the following antibodies diluted in 0.5% nonfat milk in TBS: anti-PKC-α, -β or -γ monoclonal (Upstate Biochemicals, Inc.), 1 *µ*g/ml; anti-PKC-δ or -ζ polyclonal (Gibco BKL), 1:2000 dilution; anti-PKC-∈ [Huwiler et al., *Biochem*. *J.* **1991**, 279, 441-445], 1:4000 dilution; or anti-PKC-*η*, 1:4000 dilution. Antibody incubation was followed by three washes in TBS containing 0.1% nonfat milk and the membrane was then incubated with 5 *µ*Ci ¹²⁵I-goat anti-rabbit or anti-mouse antibody (ICN Radiochemicals, Irvine CA) for one hour. Membranes were washed extensively and the labelled proteins were visualized and quantitated using a phosphorimager (Molecular Dynamics, CA).

Analysis of the PKC isozymes expressed after oligonucleotide treatment revealed that levels of PKC-δ, -∈and -ζ were unchanged by treatment with either of the antisense oligonucleotides or their scrambled controls. The antisense oligonucleotides decreased PKC-a expression by 70-80%, while scrambled controls had no effect.

### Example 10 Effect of oligonucleotides on A549 cell proliferation

A549 cells were plated in 6-well plates at a concentration of 4000 cells per well. After 24 hours cells were washed three times in DMEM and then oligonucleotides were added to the required concentration in the presence of 20 *µ*g/ml DOTMA for four hours. Cells were then washed once in DMEM plus 5% FCS and allowed to grow in DMEM plus 5% FCS for a further 72 hours. At this time cells were washed twice in PBS, removed from the dishes with trypsin and counted with a hemocytometer.

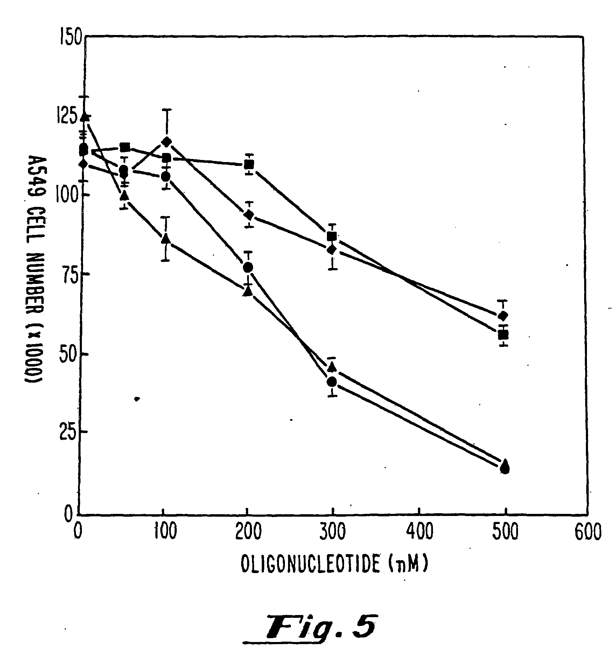

Inhibition of A549 cell proliferation by ISIS 3521 and 3527 was seen after a single oligonucleotide dose of 250-500 nM. This is shown in Figure 5.

### Example 11 Inhibition of PKCα by chimeric antisense oligonucleotides

Chimeric phosphorothioate oligonucleotides having "deoxy gaps" of 4 to 8 deoxynucleotides in an otherwise 2'-O-methyl oligonucleotide were tested for ability to decrease PKCα mRNA levels as described in Example 8. Chimeric oligonucleotides were identical in sequence to ISIS 3521 (SEQ ID NO:2) ISIS 3522 (SEQ ID NO: 3) and ISIS 3527 (SEQ ID NO:5). Chimeric oligonucleotides are shown in Table 7:

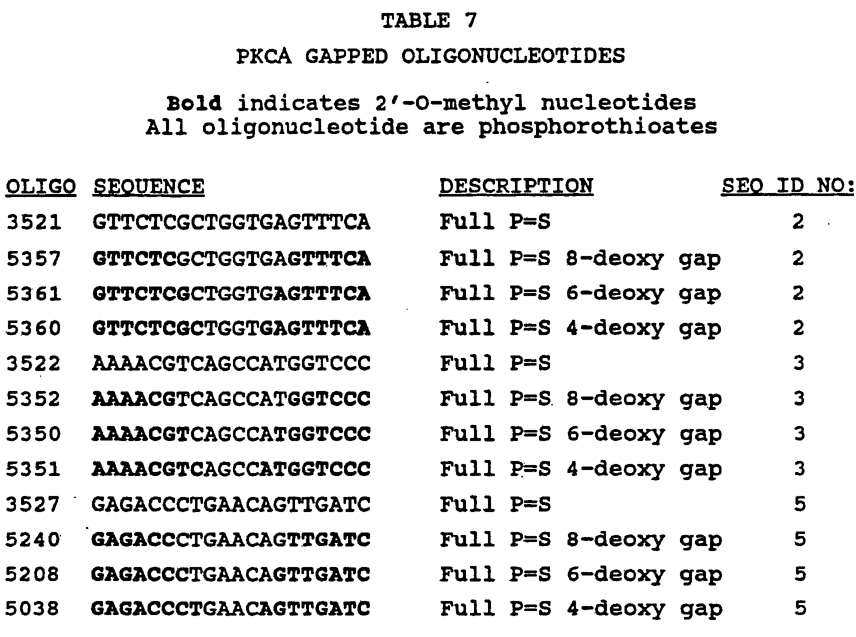

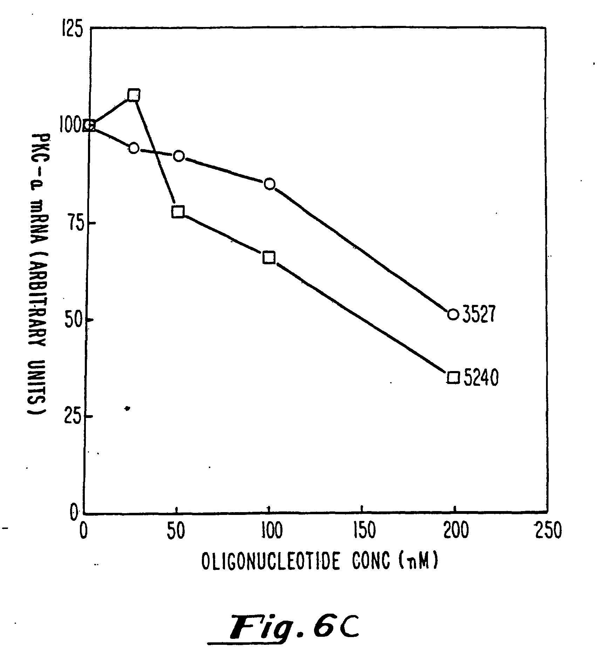

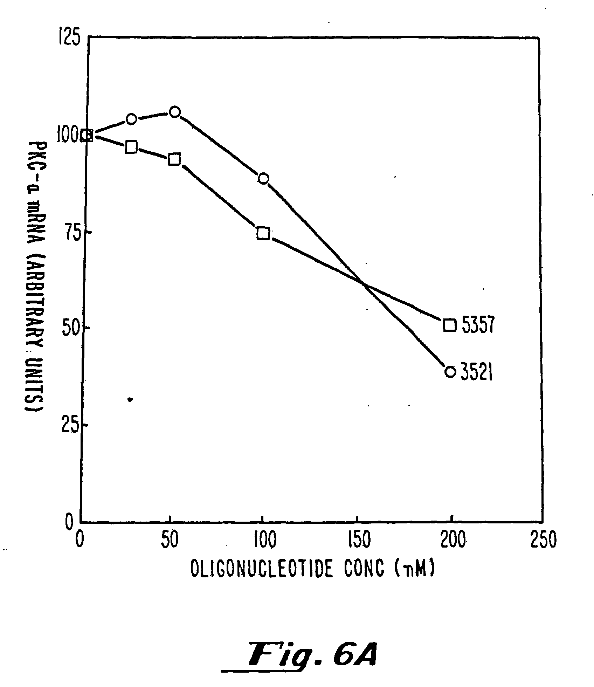

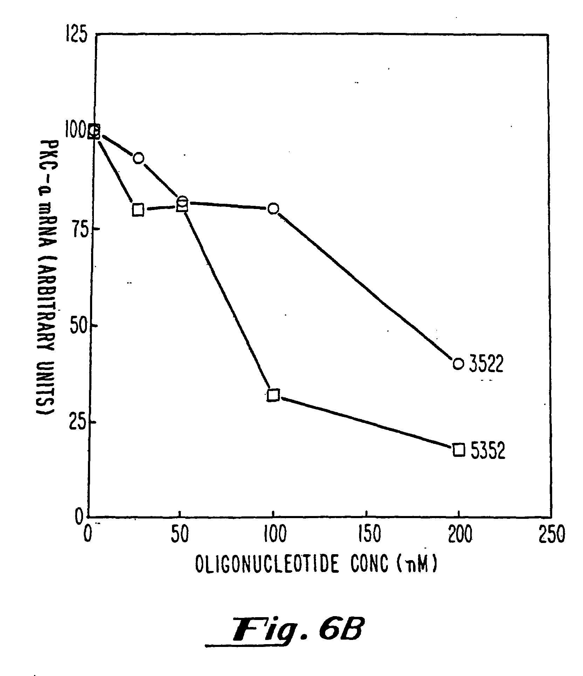

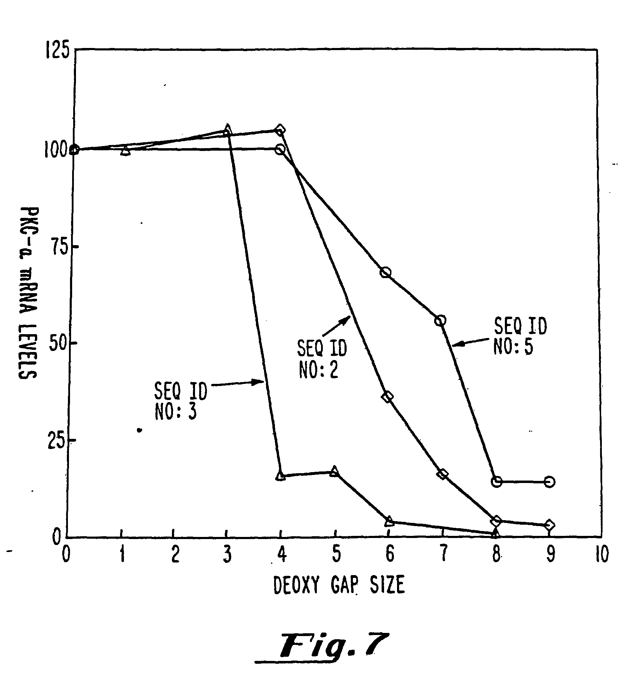

The 8-deoxy gapped oligonucleotides were able to lower PKCα mRNA levels by at least 85%. Activity of 8-deoxy gapped oligonucleotides compared to ungapped oligonucleotides having the same sequence is shown in Figure 6A, 6B and 6C. Two of the 6-deoxy gapped oligonucleotides (ISIS 5361, SEQ ID NO: 2 and ISIS 5350, SEQ ID NO:3 ) were able to lower PKCα mRNA by greater than 50%, and one of the 4-deoxy gapped oligonucleotides (ISIS 3522, SEQ ID NO: 3) was able to inhibit PKCα mRNA by approximately 85%. Activity vs. deoxy gap length for oligos having SEQ ID NO: 2, SEQ ID NO: 3 and SEQ ID NO: 5 is shown in Figure 7.

### Example 12 Oligonucleotide treatment of human tumor cells in nude mice

Human lung carcinoma A549 cells were harvested and 5 x 10⁶ cells were injected subcutaneously into the inside hind leg of nude mice. Palpable tumors develop in approximately one month. Antisense oligonucleotides ISIS 3521 and 3527 are administered to mice intraperitoneally at two doses, 1 and 10 mg/kg body weight, every other day for six weeks. Mice are monitored for tumor growth and after six weeks the tumors are excised and the expression of PKCα is determined by Northern blot and immunoblot.

## Claims

1. An oligonucleotide up to 50 nucleotide units in length which binds mRNA encoding human protein kinase C, wherein said oligonucleotide comprises a nucleotide sequence selected from the group consisting of SEQ ID NO: 6, 7, 14, 17, 18, 40, 41, 42, 43, 45 and 47.

2. The oligonucleotide of claim 1, wherein at least one of the intersugar linkages between nucleotide units of the oligonucleotide is a phosphorothioate.

3. The oligonucleotide of claim 1, wherein at least one of the nucleotides comprises a modification on the 2' position of the sugar.

4. The oligonucleotide of claim 3, wherein the modification is a 2'-O-alkyl or 2'fluoro modification.

5. The oligonucleotide of claim 4, wherein the modification is a 2'-O-methyl or 2'-O-propyl modification.

6. The oligonucleotide of claim 1 which comprises a deoxy gap region of 4 to 8 deoxynucleotides.

7. A pharmaceutical composition comprising an oligonucleotide according to any one of claims 1 to 6 and a pharmaceutically acceptable carrier.

8. An oligonucleotide according to any one of claims 1 to 6 for use in medicine.

9. Use of an oligonucleotide according to any one of claims 1 to 6 in the preparation of a composition for modulating the expression of protein kinase C in cells or tissues.

10. Use according to claim 9 for the treatment or diagnosis of conditions associated with protein kinase C.

11. A method for detecting the presence of DNA or RNA which encodes protein kinase C in cells or tissues comprising contacting the cells or tissues ex vivo with an oligonucleotide according to any one of claims 1 to 6.


SEQUENCE LISTING
(1) GENERAL INFORMATION:
(i) APPLICANT: Nicholas Dean, C. Frank Bennett
(ii) TITLE OF INVENTION: Oligonucleotide
Modulation of Protein Kinase C
(iii) NUMBER OF SEQUENCES: 51
(iv) CORRESPONDENCE ADDRESS:
(A) ADDRESSEE: Woodcock Washburn Kurtz
Mackiewicz & Norris
(B) STREET: One Liberty Place - 46th Floor
(C) CITY: Philadelphia
(D) STATE: PA
(E) COUNTRY: USA
(F) ZIP: 19103
(v) COMPUTER READABLE FORM:
(A) MEDIUM TYPE: DISKETTE, 3.5 INCH, 1.44
Mb STORAGE
(B) COMPUTER: IBM PS/2
(C) OPERATING SYSTEM: PC-DOS
(D) SOFTWARE: WORDPERFECT 5.0
(vi) CURRENT APPLICATION DATA:
(A) APPLICATION NUMBER: n/a
(B) FILING DATE: herewith
(C) CLASSIFICATION:
(vii) PRIOR APPLICATION DATA:
(A) APPLICATION NUMBER:
(B) FILING DATE:
(viii) ATTORNEY/AGENT INFORMATION:
(A) NAME: Jane Massey Licata
(B) REGISTRATION NUMBER: 32,257

(C) REFERENCE/DOCKET NUMBER: ISIS-0872
(ix) TELECOMMUNICATION INFORMATION:
(A) TELEPHONE: (215) 568-3100
(B) TELEFAX: (215) 568-3439
(2) INFORMATION FOR SEQ ID NO: 1:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 1:
CCCCAACCAC CTCTTGCTCC 20
(2) INFORMATION FOR SEQ ID NO: 2:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 2:
GTTCTCGCTG GTGAGTTTCA 20
(2) INFORMATION FOR SEQ ID NO: 3:
(i) SEQUENCE CHARACTERISTICS:
(A) LENGTH: 20
(B) TYPE: nucleic acid
(C) STRANDEDNESS: single
(D) TOPOLOGY: linear

(iv) ANTI-SENSE: yes
(xi) SEQUENCE DESCRIPTION: SEQ ID NO: 3:
AAAACGTCAG CCATGGTCCC 20
(2) INFORMATION FOR SEQ ID NO: 4:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 4:
GGATTCACTT CCACTGCGGG 20
(2) INFORMATION FOR SEQ ID NO: 5:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 5:
GAGACCCTGA ACAGTTGATC 20
(2) INFORMATION FOR SEQ ID NO: 6:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes

(xi) SEQUENCE DESCRIPTION: SEQ ID NO: 6:
CCCGGGAAAA CGTCAGCCAT 20
(2) INFORMATION FOR SEQ ID NO: 7:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 7:
CTGCCTCAGC GCCCCTTTGC 20
(2) INFORMATION FOR SEQ ID NO: 8:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 8:
AGTCGGTGCA GTGGCTGGAG 20
(2) INFORMATION FOR SEQ ID NO: 9:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 9:

GCAGAGGCTG GGGACATTGA 20

(2) INFORMATION FOR SEQ ID NO: 10:

(i) SEQUENCE CHARACTERISTICS:

(A) LENGTH: 20

(B) TYPE: nucleic acid

(C) STRANDEDNESS: single

(D) TOPOLOGY: linear

(iv) ANTI-SENSE: yes

(xi) SEQUENCE DESCRIPTION: SEQ ID NO: 10:

GGGCTGGGGA GGTGTTTGTT 20

(2) INFORMATION FOR SEQ ID NO: 11:

(i) SEQUENCE CHARACTERISTICS:

(A) LENGTH: 20

(B) TYPE: nucleic acid

(C) STRANDEDNESS: single

(D) TOPOLOGY: linear

(iv) ANTI-SENSE: yes

(xi) SEQUENCE DESCRIPTION: SEQ ID NO: 11:

CACTGCGGGG AGGGCTGGGG 20

(2) INFORMATION FOR SEQ ID NO: 12:

(i) SEQUENCE CHARACTERISTICS:

(A) LENGTH: 20

(B) TYPE: nucleic acid

(C) STRANDEDNESS: single

(D) TOPOLOGY: linear

(iv) ANTI-SENSE: yes

(xi) SEQUENCE DESCRIPTION: SEQ ID NO: 12:

AGCCGTGGCC TTAAAATTTT 20

(2) INFORMATION FOR SEQ ID NO: 13:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 13:
ATTTTCAGGC CTCCATATGG 20
(2) INFORMATION FOR SEQ ID NO: 14:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 14:
AAGAGAGAGA CCCTGAACAG 20
(2) INFORMATION FOR SEQ ID NO: 15:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 15:
GATAATGTTC TTGGTTGTAA 20

(2) INFORMATION FOR SEQ ID NO: 16:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 16:
ATGGGGTGCA CAAACTGGGG 20
(2) INFORMATION FOR SEQ ID NO: 17:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 17:
GTCAGCCATG GTCCCCCCCC 20
(2) INFORMATION FOR SEQ ID NO: 18:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 18:
CGCCGTGGAG TCGTTGCCCG 20

(2) INFORMATION FOR SEQ ID NO: 19:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 19:
TCAAATGGAG GCTGCCCGGC 20
(2) INFORMATION FOR SEQ ID NO: 20:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 20:
TGGAATCAGA CACAAGCCGT 20
(2) INFORMATION FOR SEQ ID NO: 21:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 21:
CATCTTGCGC GCGGGGAGCC 20

(2) INFORMATION FOR SEQ ID NO: 22:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 22:
TGCGCGCGGG GAGCCGGAGC 20
(2) INFORMATION FOR SEQ ID NO: 23:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 23:
CGAGAGGTGC CGGCCCCGGG 20
(2) INFORMATION FOR SEQ ID NO: 24:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 24:
CTCTCCTCGC CCTCCGTCGG 20

(2) INFORMATION FOR SEQ ID NO: 25:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 25:
TGGAGTTTGC ATTCACCTAC 20
(2) INFORMATION FOR SEQ ID NO: 26:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 26:
AAAGGCCTCT AAGACAAGCT 20
(2) INFORMATION FOR SEQ ID NO: 27:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 27:
GCCAGCATGT GCACCGTGAA 20

(2) INFORMATION FOR SEQ ID NO: 28:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 28:
ACACCCCAGG CTCAACGATG 20
(2) INFORMATION FOR SEQ ID NO: 29:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 29:
CCGAAGCTTA CTCACAATTT 20
(2) INFORMATION FOR SEQ ID NO: 30:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 30:
ACTTAGCTCT TGACTTCGGG 20

(2) INFORMATION FOR SEQ ID NO: 31:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 31:
ATGCTGCGGA AAATAAATTG 20
(2) INFORMATION FOR SEQ ID NO: 32:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 32:
ATTTTATTTT GAGCATGTTC 20
(2) INFORMATION FOR SEQ ID NO: 33:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 33:
TTTGGGGATG AGGGTGAGCA 20

(2) INFORMATION FOR SEQ ID NO: 34:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 34:
CCCATTCCCA CAGGCCTGAG 20
(2) INFORMATION FOR SEQ ID NO: 35:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 35:
CGGAGCGCGC CAGGCAGGGA 20
(2) INFORMATION FOR SEQ ID NO: 36:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 36:
CCTTTTCCCA GACCAGCCAT 20

(2) INFORMATION FOR SEQ ID NO: 37:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 37:
GGCCCCAGAA ACGTAGCAGG 20
(2) INFORMATION FOR SEQ ID NO: 38:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 38:
GGATCCTGCC TTTCTTGGGG 20
(2) INFORMATION FOR SEQ ID NO: 39:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 39:
CAGCCATGGC CCCAGAAACG 20

(2) INFORMATION FOR SEQ ID NO: 40:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 40:
CGACATGCCG GCGCCGCTGC 20
(2) INFORMATION FOR SEQ ID NO: 41:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 41:
CAGACGACAT GCCGGCGCCG 20
(2) INFORMATION FOR SEQ ID NO: 42:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 42:
   GCCTGCTTCG CAGCGGGAGA 20

(2) INFORMATION FOR SEQ ID NO: 43:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 43:
ACAGGTGCAG GAGTCGAGGC 20
(2) INFORMATION FOR SEQ ID NO: 44:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 44:
GTCCCGTCTC AGGCCAGCCC 20
(2) INFORMATION FOR SEQ ID NO: 45:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 45:
CCTCACCGAT GCGGACCCTC 20

(2) INFORMATION FOR SEQ ID NO: 46:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 46:
ATTGAACTTC ATGGTGCCAG 20
(2) INFORMATION FOR SEQ ID NO: 47:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 47:
TCTCACTCCC CATAAGGCTA 20
(2) INFORMATION FOR SEQ ID NO: 48:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 48:
TTCCTTTGGG TTCTCGTGCC 20

(2) INFORMATION FOR SEQ ID NO: 49:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 49:
TTCCATCCTT CGACAGAGTT 20
(2) INFORMATION FOR SEQ ID NO: 50:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 50:
AGGCTGATGC TGGGAAGGTC 20
(2) INFORMATION FOR SEQ ID NO: 51:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: yes
(xi) SEQUENCE DESCRIPTION: SEQ ID NO: 51:
GTTCTAAGGC TGATGCTGGG 20

(2) INFORMATION FOR SEQ ID NO: 52:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: no
(xi) SEQUENCE DESCRIPTION: SEQ ID NO: 52
TAACATACAT CATGGGGCCG 20
(2) INFORMATION FOR SEQ ID NO: 53
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: no
(xi) SEQUENCE DESCRIPTION: SEQ ID NO: 53:
GGTTTTACCA TCGGTTCTGG 20
(2) INFORMATION FOR SEQ ID NO: 54:
(i) SEQUENCE CHARACTERISTICS:
(A) LENGTH: 20
(B) TYPE: nucleic acid
(C) STRANDEDNESS: single
(D) TOPOLOGY: linear
(iv) ANTI-SENSE: no
(xi) SEQUENCE DESCRIPTION: SEQ ID NO: 54:
GTTACACAGG GACTCAACCC 20